# EUROPEAN PATENT SPECIFICATION

(11) **EP 2 460 435 B1**
(45) Date of publication and mention of the grant of the patent: **03.07.2019**
(21) Application number: 10804501.4
(22) Date of filing: 29.07.2010
(51) Int. Cl.: A45F 5/10, A45F 3/18

(54) **FACE MASK FOR COSMETIC USE AND COSMETIC METHOD**
GESICHTSMASKE ZUR KOSMETISCHEN VERWENDUNG UND KOSMETISCHES VERFAHREN
MASQUE FACIAL À USAGE COSMÉTIQUE ET PROCÉDÉ COSMÉTIQUE

(30) Priority: 31.07.2009 JP 2009179473
(43) Date of publication of application: 06.06.2012
(73) Proprietor: Shiseido Company, Ltd., Chuo-ku Tokyo 104-8010 (JP)
(72) Inventor: NASU, Mieko, Yokohama-shi, Kanagawa 224-8558 (JP); SAIWAKI, Takuya, Tokyo 104-8010 (JP); FUKUI, Hiroshi, Yokohama-shi, Kanagawa 224-8558 (JP)
(74) Representative: Santarelli
(86) International application number: PCT/JP2010/062812
(87) International publication number: WO 2011/013755

(56) References cited:
- JP-A- 8 131 253
- JP-A- 2002 000 744
- JP-A- 2005 160 833
- JP-U- H0 521 813
- US-A1- 2008 060 652

## Description

### TECHNICAL FIELD

The present invention relates to facial treatment masks and beauty care methods, and more particularly to a facial treatment mask to be attached on a human face and used for increasing a beauty care effect and to a beauty care method using the facial treatment mask.

### BACKGROUND ART

There is a recent tendency for skin care methods to diversify. A common skin care method is to directly apply a cosmetic material on skin and take care of the skin through the action of an active ingredient contained in this cosmetic material. On the other hand, it has often been used as another skin care method to cover a human face with a mask impregnated with a cosmetic material (hereinafter referred to as "cosmetic material impregnated mask"), thereby causing a beauty care ingredient of the cosmetic material to effectively penetrate into the skin. In this case, nonwoven cloth is used as the material of the cosmetic material impregnated mask.

According to this skin care method using the cosmetic material impregnated mask, it is desired to adhere the cosmetic material impregnated mask to the face in order for the cosmetic material to effectively penetrate into the skin. The presence of a gap between the face and the cosmetic material impregnated mask prevents the cosmetic material impregnated into the cosmetic material impregnated mask from penetrating into the skin.

Therefore, conventionally, it has been performed to form a cut or hole in the mask body of nonwoven cloth of a cosmetic material impregnated mask. (See, for example, Patent Documents 1 and 2.) Forming a cut or hole in the mask body makes it possible to bend the mask body at the position of its formation, thus making it possible to change the shape of the cosmetic material impregnated mask in conformation to a facial contour.

Patent document 3 discloses a face mask to be applied to the face of a person, said mask comprising a film.

### PRIOR ART DOCUMENT

### PATENT DOCUMENT

[Patent Document 1] Japanese Laid-Open Patent Application No. 2004-113511
[Patent Document 2] Japanese Laid-Open Patent Application No. 2004-262901
[Patent Document 3] Japanese Patent Application No. 2005-160833

### SUMMARY OF THE INVENTION

### PROBLEMS TO BE SOLVED BY THE INVENTION

However, the facial contour is complicated, so that there is a problem in that it is difficult to attach a cosmetic material impregnated mask onto the entire face without a gap with mere formation of a cut or hole in the mask body.

Further, if the cosmetic material impregnated into the cosmetic material impregnated mask has volatility, mere impregnation of the mask body with the cosmetic material results in the volatilization and scattering of the cosmetic material. Therefore, there is also the problem of inability to cause the cosmetic material to effectively penetrate into skin.

The present invention is made in view of the above, and has an object of providing a facial treatment mask and a beauty care method capable of causing a cosmetic material to effectively penetrate into skin.

### MEANS FOR SOLVING THE PROBLEMS

According to one aspect of the present invention, there is provided a facial treatment mask according to claims 1 and 3.

According to one aspect of the present invention, there is provided a beauty care method according to claims 4 and 5.

### EFFECTS OF THE INVENTION

A facial treatment mask according to an embodiment of the present invention includes a mask body whose interior surface has a shape conforming to the facial shape of an individual person. This enables the facial treatment mask to adhere to the face of the person without a gap.

### BRIEF DESCRIPTION OF THE DRAWINGS

Other objects, features and advantages of the present invention will become more apparent from the following detailed description when read in conjunction with the accompanying drawings, in which:
FIG. 1A is a partial cross-sectional view illustrating a state where a facial treatment mask useful for understanding the invention is attached to a person's face;
FIG. 1B is a perspective view of the facial treatment mask useful for understanding the present invention;
FIG. 2A is a diagram for illustrating a method of manufacturing the facial treatment mask useful for understanding the invention;
FIG. 2B is a diagram for illustrating the method of manufacturing the facial treatment mask useful for understanding the present invention;
FIG. 2C is a diagram for illustrating the method of manufacturing the facial treatment useful for understanding the present invention;
FIG. 2D is a diagram for illustrating the method of manufacturing the facial treatment useful for understanding the present invention;
FIG. 3A is a diagram for illustrating a beauty care method using the facial treatment mask useful for understanding the invention.
FIG. 3B is a diagram for illustrating the beauty care method using the facial treatment mask useful for understanding the present invention;
FIG. 3C is a diagram for illustrating the beauty care method using the facial treatment useful for understanding the present invention;
FIG. 4 is a front view of a cosmetic material impregnated mask not according to the embodiment of the present invention;
FIG. 5 is a cross-sectional view of a first variation of the facial treatment mask according to the embodiment of the present invention; and
FIG. 6 is a cross-sectional view of a second variation of the facial treatment mask according to the embodiment of the present invention.

### MODE FOR CARRYING OUT THE INVENTION

A description is given below, with reference to the accompanying drawings, of an embodiment of the present invention.

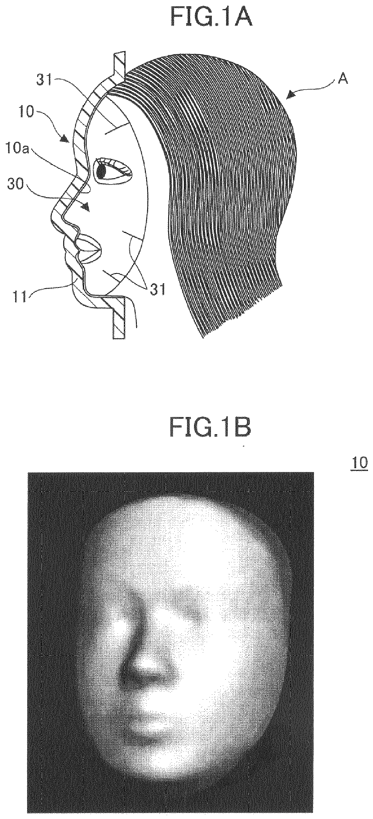

FIG. 1A illustrates a state where a facial treatment mask 10 useful for understanding the present invention is attached to a person A, and FIG. 1B illustrates the exterior of the facial treatment mask 10. As illustrated in FIG. 1A, the facial treatment mask 10 is a mask for facial treatment that is attached onto the face of the person A so as to cover the face and used.

The facial treatment mask 10 includes a mask body 11. The facial treatment mask 10 is a resin molded product formed of resin such as amorphous polyethylene terephthalate (A-PET), polyvinyl chloride (PVC), or expanded polyethylene (PE). Accordingly, the facial treatment mask 10 is impermeable to outside air. However, the facial treatment mask 10 has vents (not graphically illustrated) provided at positions opposed to the nose and the mouth of the person A, so that the person A does not have difficulty in breathing when the facial treatment mask 10 is attached on the person A.

Further, the facial treatment mask 10 (the mask body 11) according to this example is configured to have an interior surface 10a so shaped as to conform to the facial shape of the person A. That is, individual persons A have different features, so that the surfaces of their faces are different in shape (contour). According to this embodiment, the facial treatment mask 10 is manufactured in a so-called "made-to-order" manner, so that its interior surface 10a is shaped along the shape of the surface of the face of the individual person A.

As a result, when the facial treatment mask 10 is attached on the person A, the facial treatment mask 10 fits (adheres) to the face of the person A. Here, that the facial treatment mask 10 fits to the face of the person A refers to the state where the interior surface 10a of the facial treatment mask 10 and the surface of the face of the person A are in contact or a certain interval or less apart (for example, 1 mm or less, depending on the thickness of a cosmetic material impregnated mask 30 described below).

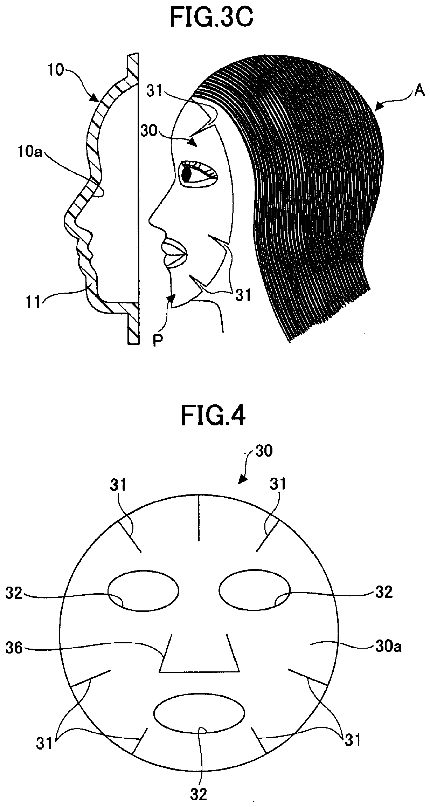

Referring to FIG. 1A, the cosmetic material impregnated mask 30, which has a mask body 30a (FIG. 4) impregnated with a cosmetic material, is placed on the face of the person A. A description is given below of this cosmetic material impregnated mask 30.

Next, a description is given of a method of manufacturing the facial treatment mask 10 having the above-described configuration.

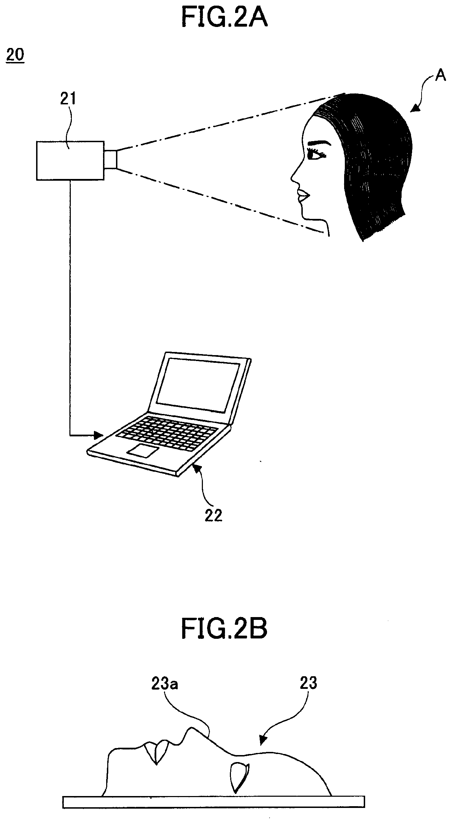

FIGS. 2A through 2D illustrates a method of manufacturing the facial treatment mask 10 along a manufacturing process. In manufacturing the facial treatment mask 10, first, the three-dimensional image data of the face of the person A are captured. A three-dimensional measurement unit 20 illustrated in FIG. 2A is used to capture the three-dimensional image data of the face of the person A. The three-dimensional measurement unit 20 includes a camera 21 and an image generator 22.

The camera 21 is a digital camera such as a CCD camera, and captures an image of the face of the person A. The data of the captured image of the person A are transmitted to the image generator 22. It is desirable that the camera 21 be higher in resolution, but may be similar in resolution to commercially available digital cameras.

According to this embodiment, the image generator 22 is implemented by a personal computer. Software for generating a three-dimensional image is installed in the personal computer. By executing this software for generating a three-dimensional image, the three-dimensional image data of the person A are generated based on the captured image data transmitted from the camera 21.

The three-dimensional image data of the person A are transmitted to a three-dimensional precision processor, which is not illustrated in the drawings. This three-dimensional precision processor, which is a multi-axis (for example, five-axis) machining center, makes a face model corresponding to the facial shape of the person A by cutting a base material based on the three-dimensional image data of the person A.

FIG. 2B illustrates the face model of the person A thus made. This face model serves as a mold in the below-described process of molding the facial treatment mask 10. (Hereinafter, the face model is referred to as "mold 23.") As is clear from the above description, the shape of a surface 23a of the mold 23 conforms to the facial shape of the person A.

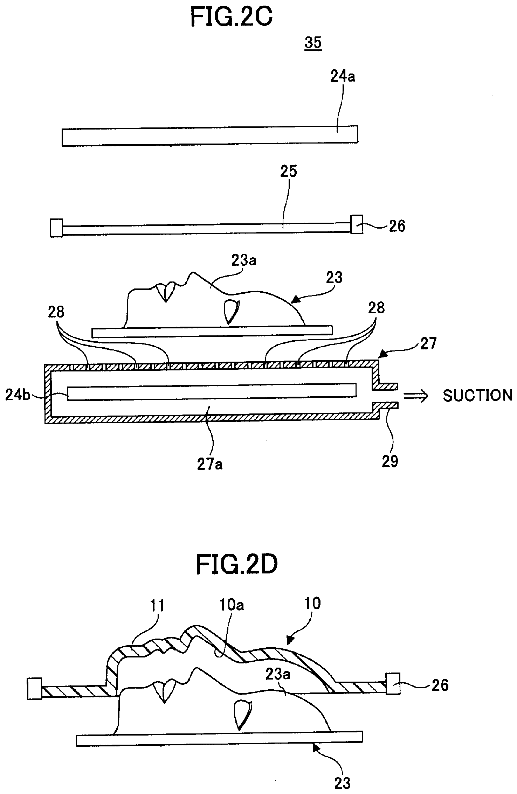

FIG. 2C illustrates the process of molding the facial treatment mask 10 using the mold 23. The molding of the facial treatment mask 10 is performed using a vacuum molding apparatus 35 and a mask material 25. The vacuum molding apparatus 35 includes the mold 23, an upper heater 24a, a lower heater 24b, and a molding base 27.

As illustrated in FIG. 2C, the mold 23 is mounted on the molding base 27. The molding base 27 has multiple suction holes 28 formed at its surface on which the mold 23 is mounted. Further, a space part 27a is formed inside the molding base 27. The suction holes 28 communicate with the space part 27a.

Further, a passage 29 formed on a side part of the molding base 27 is connected to a vacuum pump not graphically illustrated. Therefore, the starting of the vacuum pump causes the pressure inside the space part 27a to become negative and causes suction to be performed where the suction holes 28 are formed. This suction force fixes the mold 23 to the molding base 27.

The mask material 25 is a material sheet to become the facial treatment mask 10. As described above, a resin such as A-PET, PVC, or expanded PE may be used for the mask material 25. In this embodiment, A-PET, which is excellent in characteristics such as thermoformability (vacuum formability), a gas barrier characteristic, a hygienic characteristic, and chemical resistance, is used for the mask material 25.

The mask material 25 has its periphery held by a frame member 26. Further, the lower heater 24b is provided inside the space part 27a of the molding base 27 and the upper heater 24a is provided above the mask material 25. That is, the mold 23 is interposed between a pair of heaters, that is, the upper heater 24a and the lower heater 24b. The mask material 25 is heated by the paired heaters 24a and 24b to be softened.

In molding the facial treatment mask 10 using the vacuum molding apparatus 35 having the above-described configuration, first, the mold 23 is attached to the molding base 27. At this point, the vacuum pump is stopped, so that no suction force is exerted. Next, the frame member 26 is moved downward so as to place the mask member 25 on the surface 23a of the mold 23. At this point, there may be a gap formed between the surface 23a and the mask member 25.

The attachment of the mold 23 and the mask material 25 relative to the molding base 27 is thus completed, when the heaters 24a and 24b and the vacuum pump are started. As a result of the starting of the heaters 24a and 24b, the mask material 25 is heated and softened.

This example illustrates the case of starting heating the mask material 25 after the mask material 25 comes into contact with the mold 23. However, the mask material 25 may be preheated with the upper heater 24a before being placed on the mold 23. Further, a mold release agent may be applied in advance on the surface 23a of the mold 23.

The frame member 26 holding the mask material 25 is attached by suction to the molding base 27, so that the frame member 26 adheres and is fixed to the molding base 27. Then, the mask material 25 is subjected to vacuum suction by the molding base 27 inside the frame member 26, and therefore adheres to the surface 23a of the mold 23. At this point, the mask material 25, which has been heated by the heaters 24a and 24b and softened, adheres to the surface 23a of the mold 23 with no gap.

When the mask material 25 adheres to the surface 23a of the mold 23, the heaters 24a and 24b are stopped. As a result, the temperature of the mask material 25 lowers, so that the mask material 25 is cured. When the hardening of the mask material 25 is completed, the mask material 25 is released from the mold 23 as illustrated in FIG. 2D. Thereby, the facial treatment mask 10 is manufactured. The facial treatment mask 10 thus manufactured has its interior surface 10a shaped to conform to the facial shape of the person A.

According to the method of manufacturing the facial treatment mask 10 of this embodiment, the facial treatment mask 10 is manufactured using the mold 23 having the surface 23a conforming to the facial shape of the person A as well as vacuum molding. Accordingly, the facial treatment mask 10 having the interior surface 10a conforming to the facial shape of the person A is manufactured with ease and accuracy.

Further, the image generator 22 of the three-dimensional measurement unit 20 and the vacuum molding apparatus 35, which are difficult to install in the house of the person A, are installed in a manufacturing factory of the facial treatment mask 10 (hereinafter referred to as "mask making facility"). However, an image of the face of the person A, which may be captured with a commercially available digital camera as described above, may be captured at home.

As described above, the facial treatment mask 10 is manufactured to order for the individual person A. The data of the image of the person A captured with a digital camera (picture data) may be easily sent to the mask making facility through recording on a recording medium such as an SD memory card (registered trademark) or transmission using the Internet. Accordingly, it is possible to provide the individual person A with the facial treatment mask 10 with ease and certainty although the facial treatment mask 10 is custom-made.

Next, a description is given of a beauty care method using the above-described facial treatment mask 10.

FIG. 1A, FIGS. 3A through 3C, and FIG. 4 are diagrams for illustrating a beauty care method using the facial treatment mask 10 useful for understanding the present invention. The beauty care method according to this example causes a cosmetic material to penetrate into the face of the person A using two masks: the facial treatment mask 10 and the cosmetic material impregnated mask 30.

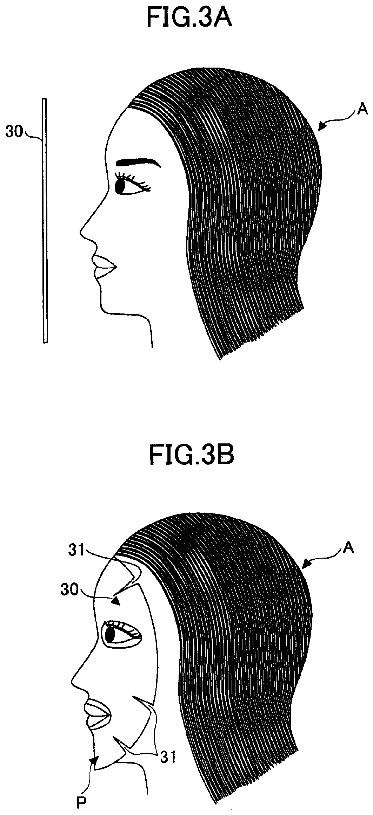

Further, the beauty care method according to this example includes a first step of attaching the cosmetic material impregnated mask 30 onto the face of the person A and a second step of further attaching the facial treatment mask 10 over the cosmetic material impregnated mask 30. FIGS. 3A and 3B illustrate the first step, and FIG. 3C and FIG. 1A illustrate the second step.

FIG. 3A illustrates a state before attaching the cosmetic material impregnated mask 30 onto the face of the person A. The cosmetic material impregnated mask 30 is formed of water-containable nonwoven cloth. The material of the cosmetic material impregnated mask 30 is not limited to nonwoven cloth, and may be water-containable woven cloth, cotton, or paper.

The cosmetic material impregnated mask 30 is impregnated with a cosmetic material. The cosmetic material with which the cosmetic material impregnated mask 30 is impregnated is formed by adding purified water, antiseptic, ethanol, etc., to active ingredients as a mask, such as a medicine, a humectant, and a beauty care ingredient. The cosmetic material has its active ingredients penetrate into skin to produce a skin care effect when adhering to the skin.

These ingredients are mixed with a ratio of, for example, 1.0% to 30% of a humectant, 0.05% to 5% of a medicine, 0.1% to 10% of a polymer, an appropriate amount of purified water, and the balance of ethanol. The active ingredients as a mask are not limited to those described above. Any ingredients may be used that can be mixed with a cosmetic material and expected to produce an effect such as a beauty care effect on skin.

FIG. 4 is a front view of the cosmetic material impregnated mask 30. The mask body 30a of the cosmetic material impregnated mask 30 has a substantially circular shape as illustrated in FIG. 4. However, the adhesion to the skin of the person A is reduced if the mask body 30a is merely circular in shape. Therefore, multiple slits 31 are formed in the mask body 30a. Further, the mask body 30a has openings 32 and a slit 36 formed at positions which are, at the time of attachment, to be opposed to the eyes and mouth and the nose, respectively, of the person A.

FIG. 3B illustrates a state where the cosmetic material impregnated mask 30 is attached to the person A. A certain beauty care effect can be expected by attaching the cosmetic material impregnated mask 30 alone. However, the attached cosmetic material impregnated mask 30, which is circular nonwoven cloth or the like provided with the slits 31, cannot have its entire surface adhere to the face of the person A, so that it is inevitable that the cosmetic material impregnated mask 30 is partly detached from the face of the person A.

In the case illustrated in FIG. 3B, the cosmetic material impregnated mask 30 is detached from the jaw part of the face of the person A. (In FIG. 3B, the detached portion of the cosmetic material impregnated mask 30 is indicated by arrow P.) This detached portion P is often generated at the jaw, near the nose, or near the lips, where the facial shape changes greatly.

Thus, in the detached portion P of the cosmetic material impregnated mask 30, the impregnated cosmetic material is prevented from penetrating into the skin of the person A, so that the beauty care effect is reduced. Further, as described above, there is a problem in that if the cosmetic material with which the cosmetic material impregnated mask 30 is impregnated is volatile, the cosmetic material volatilizes from the cosmetic material impregnated mask 30 and is therefore prevented from effectively penetrating into the skin.

Therefore, according to the beauty care method of this example, after attaching the cosmetic material impregnated mask 30 onto the face of the person A, the facial treatment mask 10 is further attached over the cosmetic material impregnated mask 30. As described above, the interior surface 10a of the facial treatment mask 10 has a shape conforming to the facial shape of the person A. Accordingly, even if there is a detached portion P in the cosmetic material impregnated mask 30, this detached portion P is pressed against the face of the person A by the facial treatment mask 10.

FIG. 1A illustrates a state where the facial treatment mask 10 is attached. As illustrated in FIG. 1A, attaching the facial treatment mask 10 makes it possible to cause the entire surface of the cosmetic material impregnated mask 30 to adhere to the face of the person A. This makes it possible to cause the cosmetic material with which the cosmetic material impregnated mask 30 is impregnated to effectively penetrate into the skin of the person A, so that it is possible to increase the beauty care effect compared with a beauty care method with the cosmetic material impregnated mask 30 only.

Further, as described above, the facial treatment mask 10 is impermeable to air. Therefore, the cosmetic material with which the cosmetic material impregnated mask 30 is impregnated is prevented from volatilizing or scattering through the facial treatment mask 10. That is, a volatile ingredient of the cosmetic material that has volatilized from the cosmetic material impregnated mask 30 (which is referred to as "volatile cosmetic ingredient") remains inside the facial treatment mask 10.

Accordingly, a volatile cosmetic ingredient, which conventionally volatilizes, also acts on the skin of the person A, which can also increase the beauty care effect. Further, compared with the conventional beauty care method, it is possible to reduce the amount of a cosmetic material for impregnating the cosmetic material impregnated mask 30.

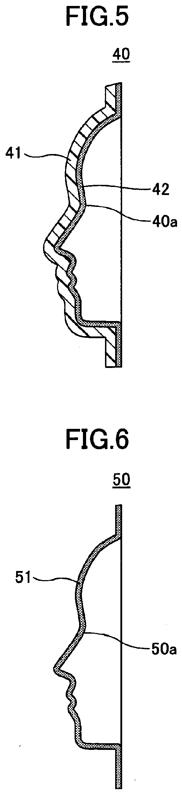

FIG. 5 and FIG. 6 illustrate variations according to the invention of the above-described facial treatment mask 10.

FIG. 5 illustrates a facial treatment mask 40 according to the invention, which is a first variation of the facial treatment mask 10. The facial treatment mask 40 according to this variation includes a mask body 41 and a deformable member 42 provided inside the mask body 41. Like the mask body 11 of the facial treatment mask 10, the mask body 41 is a resin molded product formed of resin such as A-PET, PVC, or expanded PE. Further, the deformable member 42 is formed of a deformable material such as silicon rubber or sponge. This deformable member 42 is fixed to the mask body 41 with an adhesive agent.

When the facial treatment mask 40 having the above-described configuration is attached onto the face of the person A, the deformable member 42 deforms so that an interior surface 40a of the facial treatment mask 40 deforms to a shape conforming to the facial shape of the person A. This eliminates the necessity of accurately forming the interior shape of the mask body 41 in conformity to the facial shape of the person A, thus making it possible to facilitate the manufacture of the mask body 41.

Further, by making the deformable member 42 attachable to the mask body 41 and making the deformable member 42 capable of being impregnated with a cosmetic material by, for example, forming the deformable member 42 of a material capable of being impregnated with the cosmetic material, it is possible to cause the cosmetic material to penetrate into the skin of the person A without using the cosmetic material impregnated mask 30.

FIG. 6 illustrates a facial treatment mask 50, which is a second variation of the facial treatment mask 10. The facial treatment mask 50 according to this variation has a mask body 51 formed of a deformable material. In this variation, the mask body 51 is formed of silicone rubber.

In the case of forming the entire mask body 51 of a deformable material as in this variation as well, it is possible to cause an interior surface 50a of the facial treatment mask 50 (the mask body 51) to deform into a shape further fitting the facial shape of the person A because of deformation of the mask body 51 when the facial treatment mask 50 is attached onto the face of the person A. Further, by using a water-containable material as the material of the mask body 51, it is possible to impregnate the mask body 51 with a cosmetic material.

The present invention is not limited to the specifically disclosed embodiment, and variations and modifications may be made without departing from the scope of the present invention as disclosed in the appended claims.

For example, the interior surface 10a of the facial treatment mask 10 does not have to be in perfect conformity to the facial shape of the person A, and may be locally different from the facial shape of the person A. For example, if the person A is concerned about the sag of a particular part of the facial skin, it is possible to form part of the interior surface 10a corresponding to the facial part where skin sagging occurs into a shape capable of lifting up the skin. This configuration makes it possible to simultaneously produce a beauty care effect due to the penetration of a cosmetic material into the skin and a beauty care effect to due to a lift-up of the skin.

The present application is based on and claims the benefit of priority of Japanese Patent Application No. 2009-179473, filed on July 31, 2009.

### DESCRIPTION OF THE REFERENCE NUMERALS

- 10, 40, 50: facial treatment mask
- 10a: interior surface
- 11, 41, 51: mask body
- 20: three-dimensional measurement unit
- 21: camera
- 22: image generator
- 23: mold
- 25: mask material
- 27: molding base
- 28: suction hole
- 30: cosmetic material impregnated mask
- 31: slit
- 32: opening
- 35: vacuum molding apparatus
- 42: deformable member
- A: person
- P: detached portion

## Claims

1. A facial treatment mask (40) to be attached to a face of a person so as to cover the face, the facial treatment mask (40) comprising:
a mask body (41) having an interior surface (40a) of a shape conforming to a shape of the face of the person, wherein the mask body is a resin molded cured product; and
a deformable member (42) provided on the interior surface (40a) of the mask body (41),
wherein the deformable member (42) is formed of a material capable of being impregnated with a cosmetic material.

2. The facial treatment mask as claimed in claim 1, wherein the mask body is formed of a material impermeable to air.

3. A facial treatment mask (50) to be attached to a face of a person so as to cover the face, the facial treatment mask (50)
comprising:
a mask body (51) having an interior surface (50a) of a shape conforming to a shape of the face of the person,
wherein the mask body (51) is formed of a deformable water-containable material.

4. A beauty care method, comprising the steps of:
impregnating a deformable member (42) of a facial treatment mask (40) with a cosmetic material, the facial treatment mask (40) including: a mask body (41) having an interior surface (40a) of a shape conforming to a shape of a face of a person to whom the facial treatment mask (40) is to be attached, the mask body (41) being a resin molded cured product; and the deformable member (42) provided on the interior surface (41a) of the mask body (41) and formed of a material capable of being impregnated with the cosmetic material; and
attaching the facial treatment mask (40) onto the face of the person.

5. A beauty care method, comprising the steps of:
impregnating a mask body (51) of a facial treatment mask (50) with a cosmetic material, the mask body (51) having an interior surface (50a) of a shape conforming to a shape of a face of a person to whom the facial treatment mask (50) is to be attached, the mask body (51) being formed of a deformable and water-containable material; and
attaching the facial treatment mask (40) onto the face of the person.

6. The beauty care method as claimed in claim 4 or 5, wherein the cosmetic material is volatile.

7. The beauty care method as claimed in claim 4, wherein the mask body is formed of a material impermeable to air.

## Patentansprüche

1. Gesichtsbehandlungsmaske (40), welche auf ein Gesicht einer Person zwecks Abdeckung des Gesichts aufgebracht werden soll, wobei die Gesichtsbehandlungsmaske (40) umfasst:
einen Maskenkörper (41) mit einer Innenfläche (40a), deren Form mit einer Form des Gesichts der Person übereinstimmt, wobei es sich bei dem Maskenkörper um ein gehärtetes Harzformprodukt handelt; und
ein verformbares Element (42), welches auf der Innenfläche (40a) des Maskenkörpers (41) bereitgestellt ist,
wobei das verformbare Element (42) aus einem Material gebildet ist, das mit einem kosmetischen Material imprägniert werden kann.

2. Gesichtsbehandlungsmaske gemäß Anspruch 1, wobei der Maskenkörper aus einem für Luft undurchlässigen Material gebildet ist.

3. Gesichtsbehandlungsmaske (50), welche auf ein Gesicht einer Person zwecks Abdeckung des Gesichts aufgebracht werden soll, wobei die Gesichtsbehandlungsmaske (50) umfasst:
einen Maskenkörper (51) mit einer Innenfläche (50a), deren Form mit einer Form des Gesichts der Person übereinstimmt,
wobei der Maskenkörper (51) aus einem verformbaren, wasserhaltigen Material gebildet ist.

4. Schönheitspflegeverfahren, umfassend die Schritte:
Imprägnieren eines verformbaren Elements (42) einer Gesichtsbehandlungsmaske (40) mit einem kosmetischen Material, wobei die Gesichtsbehandlungsmaske (40) umfasst: einen Maskenkörper (41) mit einer Innenfläche (40a), deren Form mit einer Form eines Gesichts einer Person übereinstimmt, auf welche die Gesichtsbehandlungsmaske (40) aufgebracht werden soll, wobei es sich bei dem Maskenkörper (41) um ein gehärtetes Harzformprodukt handelt; und das verformbare Element (42), welches auf der Innenfläche (41a) des Maskenkörpers (41) bereitgestellt ist und aus einem Material gebildet ist, das mit dem kosmetischen Material imprägniert werden kann; und
Aufbringen der Gesichtsbehandlungsmaske (40) auf das Gesicht der Person.

5. Schönheitspflegeverfahren, umfassend die Schritte:
Imprägnieren eines Maskenkörpers (51) einer Gesichtsbehandlungsmaske (50) mit einem kosmetischen Material, wobei der Maskenkörper (51) eine Innenfläche (50a) aufweist, deren Form mit einer Form eines Gesichts einer Person übereinstimmt, auf welche die Gesichtsbehandlungsmaske (50) aufgebracht werden soll, und wobei der Maskenkörper (51) aus einem verformbaren und wasserhaltigen Material gebildet ist; und
Aufbringen der Gesichtsbehandlungsmaske (40) auf das Gesicht der Person.

6. Schönheitspflegeverfahren gemäß Anspruch 4 oder 5, wobei das kosmetische Material flüchtig ist.

7. Schönheitspflegeverfahren gemäß Anspruch 4, wobei der Maskenkörper aus einem für Luft undurchlässigen Material gebildet ist.

## Revendications

1. Masque de traitement pour le visage (40) destiné à être fixé sur le visage d'une personne afin de recouvrir le visage, le masque de traitement pour le visage (40) comprenant :
un corps de masque (41) ayant une surface intérieure (40a) d'une forme se conformant à la forme du visage de la personne, dans lequel le corps de masque est un produit durci moulé en résine ; et
un élément déformable (42) prévu sur la surface intérieure (40a) du corps de masque (41),
dans lequel l'élément déformable (42) est formé d'un matériau pouvant être imprégné avec un produit cosmétique.

2. Masque de traitement pour le visage selon la revendication 1, dans lequel le corps de masque est formé d'un matériau imperméable à l'air.

3. Masque de traitement pour le visage (50) destiné à être fixé sur le visage d'une personne afin de recouvrir le visage, le masque de traitement pour le visage (50) comprenant :
un corps de masque (51) ayant une surface intérieure (50a) d'une forme se conformant à la forme du visage de la personne,
dans lequel le corps de masque (51) est formé d'un matériau déformable pouvant contenir de l'eau.

4. Procédé de soin de beauté comprenant les étapes suivantes :
imprégner un élément déformable (42) d'un masque de traitement pour le visage (40) avec un produit cosmétique, le masque de traitement pour le visage (40) comprenant : un corps de masque (41) ayant une surface intérieure (40a) d'une forme se conformant à la forme du visage d'une personne sur laquelle le masque de traitement pour le visage (40) doit être fixé, le corps de masque (41) étant un produit durci moulé en résine ; et l'élément déformable (42) prévu sur la surface intérieure (41a) du corps de masque (41) et formé d'un matériau pouvant être imprégné avec le produit cosmétique ; et
fixer le masque de traitement pour le visage (40) sur le visage de la personne.

5. Procédé de soin de beauté comprenant les étapes suivantes :
imprégner un corps de masque (51) d'un masque de traitement pour le visage (50) avec un produit cosmétique, le corps de masque (51) ayant une surface intérieure (50a) d'une forme se conformant à la forme du visage d'une personne sur lequel le masque de traitement pour le visage (50) doit être fixé, le corps de masque (51) étant formé d'un matériau déformable et pouvant contenir de l'eau ; et
fixer le masque de traitement pour le visage (40) sur le visage de la personne.

6. Procédé de soin de beauté selon la revendication 4 ou 5, dans lequel le produit cosmétique est volatil.

7. Procédé de soin de beauté selon la revendication 4, dans lequel le corps de masque est formé d'un matériau imperméable à l'air.
